Europäisches Patentamt

European Patent Office

Office européen des brevets

(11) Veröffentlichungsnummer: **0 204 989 B1**

# EUROPÄISCHE PATENTSCHRIFT

(45) Veröffentlichungstag der Patentschrift: **25.03.92**

(51) Int. Cl.⁵: **A61K 31/195**

(21) Anmeldenummer: **86106646.2**

(22) Anmeldetag: **15.05.86**

(54) **Verwendung von Penicillamin zur Behandlung von Immunmangelkrankheiten.**

(30) Priorität: **08.06.85 DE 3520624**

(43) Veröffentlichungstag der Anmeldung:
**17.12.86 Patentblatt 86/51**

(45) Bekanntmachung des Hinweises auf die
Patenterteilung:
**25.03.92 Patentblatt 92/13**

(84) Benannte Vertragsstaaten:
**AT BE CH DE FR GB IT LI LU NL SE**

(56) Entgegenhaltungen:

**Concours medical, Band 100, 23. Dezember
1978, Seiten 7845-7850 Paris, FR V. Lemaire:
"Les traitements à visée immunologique en
rhumatologie" *Seite 7850:
"Immuno-stimulation"***

**Int. Journal of Immunotherapy, Band I, Nr. 2,
1985, Seiten 79-83 Bioscience Ediprint Inc.,
CH Y. Shiokawa:"Progress in new immuno-
modulator research"*Ganze Dokument, Insbes. Tabelle IV***

(73) Patentinhaber: **ASTA Medica Aktiengesellschaft
Weismüllerstrasse 45
W-6000 Frankturt am Main 1(DE)**

(72) Erfinder: **Chandra, Prakash, Prof. Dr. phil. nat.
Breitlacher Strasse 45A
W-6000 Frankturt 90(DE)**

EP 0 204 989 B1

EP 0 204 989 B1

Postgraduate Med. J., Suppl., Band 50, 1974,
Seiten 50-55, London, GB I.A. JAFFE et al.:
"Further studies of the anti-viral effect of
D-penicillamine" *Zusammenfassung;
Introduction*

NOUVELLE PRESSE MEDICALE, Band 1, Nr.
14, Seiten 953-958, 1972, Paris, FR G.H. WER-
NER: "La multiplication des virus. 2. Perspectives d'avenir de la chimiothèrapie antivirale" *Seiten 953-954; conclusions "*

Boletino Soc. Italiana Biologica Sperimentale, Band 43, 1967, Seiten 645-647, Napoli, IT
P. MAZZUCATO et al.: "Attivita antivirale della guanidina E della D-penicillamina. II). Studio comparato degli antagonisti della guanidina E della D-penicillamina" "Seite 645; Figur 2*

Arzneimittelforsch./Drug Res.,Band 36, Nr.2,
Februar 1986, Aulendorf, DE P.CHANDRA et
al.: "Selective inhibition of replication of the
AIDS-associated virus HTLV-III/LAV by synthetic D-penicillamine" "Ganze Dokument*

THE MERCK MANUAL OF DIAGNOSIS AND
THERAPY, R. Berkow, M.D. Editor: 14. Auflage, 1982, MSD Research Lab. Rahway, NJ. US
*Seiten 288-304*

**Beschreibung**

Penicillamin ist eine unphysiologische Aminosäure, nämlich ein Dimethyl-Derivat des Cysteins. Penicillamin kann in zwei enantiomeren Formen vorkommen. Eine enantiomere Form, das D-Penicillamin, kann aus natürlichem Penicillin durch Hydrolyse oder vollsynthetisch hergestellt werden.

Das vollsynthetische D-Penicillamin kann zum Beispiel durch Racemat-Spaltung von D,L-Penicillamin mit Hilfe optisch aktiver Basen wie zum Beispiel Brucin, d-Pseudoephedrin oder l-Ephedrin (siehe "The Chemistry of Penicillin" 1949 Princeton University press; vergleiche britisches Patent 585 413, amerikanisches Patent 2 450 784, belgisches Patent 7 385 207) oder l-Norephedrin (deutsches Patent 21 38 122) erhalten werden.

Ein Vorteil des D-Penicillamins gegenüber anderen SH-Verbindungen auch gegenüber anderen Cystein-Derivaten, ist seine relative Stabilität im Stoffwechsel, wodurch seine Wirkung gut zur Entfaltung kommt.

D-Penicillamin wird seit ca. 1960 in der Therapie von verschiedenen Krankheiten eingesetzt, so zum Beispiel bei der progredient chronischen Polyarthritis, Schwermetallvergiftungen, chronisch-aggressiver Hepatitis, Leberzirrhose, Cystinurie, Cystinsteine, Sklerodermie, Morbus Wilson, Morbus Waldenström, schizophrener Defektzustand, arteriosklerotischen Erkrankungen, Lupus erythematoden und Fibrosen verschiedener Genese.

Ein spezifischer anti-viraler Effekt von D-Penicillamin gegen Poliovirus ist von I. A. Jaffe et al. Postgraduate Medical Journal, Band 90, 1974, Seiten 50 - 55, London (GB) beschrieben. Bei anderen Viren wie zum Beispiel Herpes Simplex und Vaccinia ist D-Penicillamin an Gewebekulturen unwirksam.

Es wurde nun gefunden, daß D- und L-Penicillamin ebenso wie das D,L-Racemat auch zur Therapie von Krankheiten eingesetzt werden können, die sich durch ein Immunmangelsyndrom auszeichnen. Eine Erkrankung mit fortschreitender schwerer Immundefizienz, die über die Entwicklung von Tumoren und Infektionen zum Tode führt, ist das Acquired Immune Deficiency Syndrome (AIDS). Die Erkrankung wurde erstmals 1981 erkannt, und inzwischen konnte eine virale Genese nachgewiesen werden. Auf der Suche nach der Krankheitsursache des Immunmangelsyndroms wurde eine Störung der Immunregulation und Immunabwehr gefunden. Das Verhältnis von $T_4$ (Helferzellen) zu $T_8$ (Suppressorzellen) ist gestört. In den Jahren 1983 konnte der Lymphadenopathie-Virus (LAV-I) und im Jahre 1984 konnte Human-T-cell-leukemia-Virus, das HTLV-III-Virus, ein Virus der Retroviren-Gruppe, isoliert und als Ursache von AIDS nachgewiesen werden. Der LAV-I-Virus und der HTLV-III-Virus wurden von zwei verschiedenen Forschergruppen gefunden und werden als praktisch gleich angesehen. Die Zielzellen des AIDS-Virus sind Zellen des Immunsystems. Die Infektion bleibt Monate bis Jahre inapparent bis schließlich Symptome auftreten, die zunächst unspezifisch erscheinen, aber in ihrer Kombination und langen Persistenz, zusammen mit einer häufig bestehenden Lymphadenopathie, ein deutlicher Hinweis auf eine Infektion mit diesem Virus sein können. Im weiteren Verlauf kann es zu schweren Funktionsstörungen der zellulären Immunabwehr kommen.

Als Folge treten Infektionen mit opportunistischen Erregern und/oder Tumore, wie zum Beispiel Kaposi-Sarkome und Non-Hodgkin-Lymphome auf. Die Infektionen mit opportunistischen Erregern, Parasiten und/oder das Auftreten von Tumoren bestimmen Verlauf und Ausgang der AIDS-Erkrankung. Patienten in diesem Stadium versterben innerhalb von 36 Monaten zu über 80% an diesen Komplikationen.

Folgende Personen haben ein erhöhtes Risiko, an AIDS zu erkranken: Männliche Homosexuelle mit häufig wechselnden Intimpartnern, Abhängige von i.v. verabfolgten Suchtmitteln (Fixer), Heterosexuelle Intimpartner von Infizierten und Erkrankten, Einwanderer oder Reisende aus Haiti, der Karibik oder Äquatorialafrika (z.B. Zaire), Hämophiliepatienten, die Konzentrate von Gerinnungsfaktoren (z.B. Faktor VIII) erhalten, Neugeborene AIDS-infizierter Mütter, Empfänger von AIDS-Virus haltigem Blut.

Es wurde nun in vitro gefunden, daß Penicillamin die Virus-Replikation ausgezeichnet inhibieren kann und zudem keine Toxizität gegenüber dem normalen Zellwachstum zeigt. Die Inhibierung zeigt sich sowohl mit dem D- und L-Penicillamin als auch mit dem D,L-Racemat. Bei einer Konzentration von 20 $\mu$g/ml wird die HTLV-III-Virus (LAV-I-Virus) Replikation in einer Zellkultur mit L-Penicillamin zu ca. 95% und mit D-Penicillamin zu ca. 80% gehemmt. Bei einer Konzentration von 40 $\mu$g/ml ist die Wirksamkeit sowohl von L-Penicillamin als auch von D-Penicillamin in vitro nahezu 100%.

Es ist bekannt, daß L-Penicillamin und das D,L-Racemat eine höhere Toxizität aufweisen, so daß für die Anwendung am Menschen bevorzugt das D-Enantiomere in Frage kommt.

Mit dem Medikament können natürlich nicht nur Patienten mit klinischen AIDS-Symptomen behandelt werden. Auch bereits infizierte Patienten, in deren Blut entsprechende Antikörper nachgewiesen wurden, ohne schon das Krankheitsbild zu zeigen, können in gleicher Weise behandelt werden.

Die Arzneimittel, die Penicillamin oder auch Mischungen desselben mit anderen pharmazeutisch wirksamen Stoffen enthalten, sowie gegebenenfalls Zusätze weiterer pharmazeutischer Trägermittel, können enteral, parenteral, oral, lokal, perlingual oder auch in Form von Sprays angewandt werden.

Die Verabreichung kann zum Beispiel in Form von Tabletten, Kapseln, Pillen, Dragees, Zäpfchen, Salben, Gelees, Puder, Liquida, oder Aerosolen erfolgen. Als Liquida kommen zum Beispiel in Frage: Ölige oder wässrige Lösungen oder Suspensionen, Emulsionen, injizierbare wässrige oder ölige Lösungen oder Suspensionen.

Insbesondere kommen beispielsweise folgende Arzneiformen in Betracht:

a) Orale Arzneiformen wie Granulate, Tabletten, Dragées und Kapseln, sowie Lösungen, Emulsionen und Suspensionen. Hierbei beträgt die Dosierung an D-Penicillamin zum Beispiel 125 mg, 250 mg, 300 mg oder 500 mg pro Einzeldosis.

b) Parenterale Arzneiformen zum Beispiel zur intravenösen oder intramuskulären Injektion mit beispielsweise einer Wirkstoffdosierung von 50 bis 2000 mg pro Einzeldosis.

Penicillamin kann hierbei beispielsweise in Form von D-Penicillaminhydrochlorid und/oder D-Penicillaminparatoluolsulfonat vorliegen.

c) Arzneiformen zur rektalen und vaginalen Applikation. Dosierung zum Beispiel von 50 bis 1000 mg pro Einzeldosis.

Die Herstellung der Arzneimittel kann unter Verwendung der bekannten und üblichen pharmazeutischen Träger-und Verdünnungsmittel sowie sonstiger üblicher Hilfsstoffe erfolgen. Derartige Träger- und Hilfsstoffe sind zum Beispiel in Ullmanns Encyklopädie der technischen Chemie, Band 4 (1953), Seite 1 bis 39; Journal of Pharmaceutical Sciences, Band 52 (1963), Seite 918 und ff.; H.v. Czetsch-Lindenwald, Hilfsstoffe für Pharmazie und angrenzende Gebiete; H.P. Fiedler: Lexikon der Hilfsstoffe für Pharmazie, Kosmetik und angrenzende Gebiete, Editio Cantor K.G., Aulendorf i. Württ., 1971 sowie in Pharm. Ind., Heft 2, 1961, Seite 72 und ff. angegeben.

Beispiele hierfür sind Gelatine, Rohrzucker, Pektin, Stärke, Tylose, Talkum, Lycopodium, Kieselsäure, Milchzucker, Cellulosederivate, Stearate, Emulgatoren, pflanzliche Öle, Wasser, pharmazeutisch verträgliche ein- oder mehrwertige Alkohole und Polyglykole wie Polyäthylenglykole sowie Derivate hiervon, Dimethylsulfoxyd, Ester von aliphatischen gesättigten oder ungesättigten Fettsäuren mit ein- oder mehrwertigen Alkoholen wie Glykolen, Glycerin, Diäthylenglykol, Pentaerythrit, Sorbit und Mannit, die gegebenenfalls auch veräthert sein können, Benzylbenzoat, Dioxolane, Glyzerinformale, Glycolfurole, Dimethylacetamid, Lactamide, Lactate und Äthylcarbonate.

Bei der Herstellung von Lösungen kann es erforderlich sein, zur Erzielung der gewünschten D-Penicillamin-Konzentration organische Lösungsmittel allein oder in Mischung mit Wasser einzusetzen. Als physiologisch verträgliche organische Lösungsmittel können zum Beispiel ein- oder mehrwertige Alkohole wie Äthanol, Isopropanol, Butanol, Äthylenglykol, Propylenglykole, Glycerin, Diglycerin, Triglycerin, Polyglycerine (aus 4 bis 12 Glycerineinheiten) Polyglykole wie Polyäthylenglykole, Polypropylenglykole deren Äther mit niederen aliphatischen Alkoholen sowie deren Ester mit niederen aliphatischen Carbonsäuren, aliphatische Carbonsäureamide (1 bis 10 C-Atome), N-alkylsubstituierte Carbonsäureamide wie Dimethylformamid oder Dimethylacetamid Anwendung finden.

Darüberhinaus ist der Zusatz von Konservierungsmitteln, Stabilisatoren, Puffersubstanzen, Geschmackskorrigenzien, Antioxydantien und Komplexbildnern (zum Beispiel Äthylendiaminotetraessigsäure: siehe auch U.Olthoff und R. Hüttenrauch, Die Pharmazie 26/4, 217 (1971) möglich. Gegebenenfalls ist zur Stabilisierung des D-Penicillamins mit physiologisch verträglichen Säuren oder Puffern auf einem pH-Bereich von ca. 4,0 - 4,5 einzustellen.

Als Antioxydantien kommen beispielsweise Natriummetabisulfit, als Konservierungsmittel beispielsweise Sorbinsäure, und p-Hydroxybenzoesäureester in Betracht. Der Zusatz von Carbonylverbindungen ist im allgemeinen nicht zweckmäßig.

Die pharmakologische und galenische Handhabung der erfindungsgemäßen Verbindungen erfolgt nach den üblichen Standardmethoden (siehe zum Beispiel Hagers Handbuch der Pharmazeutischen Praxis, Vierte Neuausgabe, VII Band Teil A: Arzneiformen).

Insbesondere ist auch der Zusatz anderer gegen D-Penicillamin inerter Arzneimittelwirkstoffe, vor allem von Analgetica, Antihistaminica, Antiphlogistica, Spasmolytica, Geriatrica, Lebertherapeutica, Vitamine, Spurenelemente und Steroiden möglich beziehungsweise günstig.

Vorzugsweise sollen die zusätzlichen Stoffe keine eigene optische Aktivität besitzen, da dadurch die Kontrolle des Drehwerts des D-Penicillamins erleichtert wird.

Die pharmazeutischen Zubereitungen enthalten im allgemeinen zwischen 0,5 bis 100 Gewichtsprozent D-Penicillamin.

Beispielsweise können 4 mal täglich 1 bis 6 Tabletten, vorzugsweise 2 bis 4 Tabletten, mit einem Gehalt von 125 mg bis 500 mg, vorzugsweise 300 mg, wirksamer Substanz*, empfohlen werden. Die

*Handelspräparat    Trolovol®    Bayer/Degussa    Pharma    Gruppe

Dosierung sollte zu Beginn der Behandlung einschleichend erfolgen und nach ca. 2 Wochen je nach medizinischen Erfordernissen erhöht werden. Bei intravenöser Injektion kann 1-2 mal täglich eine Ampulle von 10 ml Inhalt mit 1000 mg Substanz empfohlen werden. Es ist aus Untersuchungen bekannt, daß D-Penicillamin an Plasmaproteine (vorwiegend Albumin) gebunden wird. Da die Konzentration des freien, d.h. nicht proteingebundenen D-Penicillamin mit steigender Dosis offenbar zunimmt, ergibt sich für die therapeutische Praxis die Forderung einer nicht zu niedrigen Dosierung. Das Medikament wird nach oraler Gabe innerhalb 2 bis 3 Stunden zu rund 60% resorbiert. Es verteilt sich, wie auch andere Aminosäuren, relativ schnell über den gesamten Organismus, und die nicht ausgeschiedene Fraktion weist eine Halbwertszeit von 75 bzw. 90 Stunden auf. Die Ausscheidung erfolgt vorwiegend über die Nieren, größtenteils als Disulfid, zu 10% in unveränderter Form.

Die akute Toxizität des D-Penicillamins an der Maus (ausgedrückt durch die LD 50 mg/kg; Methode nach Miller und Tainter: Proc.Soc.Exper.Biol. a.Med. 57 (1944) 261) liegt beispielsweise bei oraler Applikation zwischen 7000 und 10500 mg/kg.

Anstelle der D-Penicillamin-Base können auch die mittels der üblichen Methoden erhaltenden Salze verwendet werden. Als Säurekomponente für die Salze kommen die üblichen pharmakologisch verwendbaren Säuren, wie zum Beispiel Salzsäure, Bromwasserstoffsäure, Schwefelsäure, Essigsäure, Zitronensäure, Bernsteinsäure, Maleinsäure, Fumarsäure, Milchsäure und Paratoluolsulfonsäure in Betracht.

Insbesondere kommen beispielsweise die Anionen folgender Säuren in Frage: HBr, HCl, HJ, $HNO_3$, $H_2SO_4$ ($SO_4^{--}$); $H_3PO_4$ ($HPO_3^{--}$); Kampfersulfonsäure, aliphatische oder aromatische Sulfonsäuren, beispielsweise $C_1$-$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure); aliphatische $C_2$-$C_4$-Monocarbonsäuren, die gegebenenfalls ein-, zwei- oder dreifach durch Halogenatome (insbesondere Cl, F) substituiert sind (zum Beispiel Essigsäure, Propionsäure, Chloressigsäure, Dichloressigsäure, Trifluoressigsäure, Trichloressigsäure); aliphatische $C_2$-$C_{11}$-Dicarbonsäuren, die gegebenenfalls eine Doppelbindung enthalten (zum Beispiel Oxalsäure, Malonsäure, Malonsäure, welche in 2-Stellung durch eine oder zwei $C_1$-$C_4$-Alkylgruppen substituiert ist, Maleinsäure, Fumarsäure, Bernsteinsäure); aliphatische Monohydroxy- und Dihydroxy-Monocarbonsäuren mit 2 bis 6, insbesondere 2 bis 3 Kohlenstoffatomen, wobei es sich vorzugsweise um $\alpha$-Monohydroxycarbonsäuren handelt wie Milchsäure, Glycerinsäure oder Glykolsäure; aliphatische Monohydroxy- und Dihydroxy-Di- und Tricarbonsäuren mit 3 bis 8 Kohlenstoffatomen, insbesondere 3 bis 6 Kohlenstoffatomen wie Tartronsäure, Äpfelsäure, Weinsäure, Malonsäure, die an dem mittelständigen C-Atom durch eine Hydroxygruppe und gegebenenfalls eine $C_1$-$C_4$-Alkylgruppe substituiert ist, Isozitronensäure oder Zitronensäure; Phthalsäure, die gegebenenfalls durch eine Carboxygruppe (insbesondere in 4-Stellung) substituiert ist; Gluconsäure; Glucuronsäure; 1,1-Cyclobutandicarbonsäure; Organophosphorsäuren, wie Aldose- und Ketosephosphorsäuren (beispielsweise die entsprechenden Mono-und Diphosphorsäuren) zum Beispiel Aldose-6-phosphorsäuren wie D- oder L-Glucose-6-phosphorsäure, $\alpha$-D-Glucose-1-phosphorsäure, D-Fructose-6-phosphorsäure, D-Galactose-6-phosphorsäure, D-Ribose-5-phosphorsäure, D-Fructose-1, 6-diphosphorsäuren; Glycerin-phosphorsäuren (wobei der Phosphorsäurerest an einem der endständigen oder an dem mittelständigen Glycerinsauerstoffatom gebunden ist) wie $\alpha$-D,L-Glycerinphosphorsäure, $\beta$-Glycerinphosphorsäure; N-Phosphono-acetyl-Asparaginsäure (zum Beispiel L-Asparaginsäure).

Vor Beginn der Behandlung mit D-Penicillamin sind Blutbild- und Urinuntersuchungen zu empfehlen.
Während der Therapie sind in bekannter Weise entsprechende medizinische Kontrolluntersuchungen vorzunehmen.

Die Wirkung der Behandlung läßt sich neben der Besserung der klinischen Symptome insbesondere durch die Erfassung der immunologischen Parameter (Verhältnis $T_4$ Helferzellen zu $T_8$ Suppressorzellen) erkennen.

Pharmazeutische Zubereitungen mit vollsynthetischem D-Penicillamin sind zum Beispiel in dem britischen Patent 1 424 432 beschrieben.

Versuchsdurchführung:

HTLV-III-Virusinfektion von H9 Zellen (Menschliche T-Zellinie von einem Leukämiepatienten vom National Cancer Institute Bethesda Maryland USA): H9 Zellen werden mit Polybrene (2ug/ml) 30 Minuten bei 37°C behandelt, anschließend wird Polybrene ausgewaschen und die Zellen werden mit $2 \times 10^8$ HTLV-III Viruspartikeln pro $4 \times 10^5$ H9 Zellen infiziert. Vor der Infizierung wird das Virus mit der Substanz bei verschiedenen Konzentrationen 45 Minuten bei 37°C inkubiert. Zur Kontrolle wird das Virus unter den gleichen experimentellen Bedingungen inkubiert, jedoch ohne Substanzzugabe. Die Zellkulturen werden am 4. Tag nach der Infizierung wie folgt analysiert:

Immunfloreszenz-Analyse: Die Wirkung von D- und L-Penicillamin auf die Vermehrung des HTLV-III-Virus in H9 Zellen wird durch die Messung der aus HTLV-III isolierten Proteinen p15 und p24 (Molekulargewicht 15000 beziehungsweise 24000) bestimmt. Die Immunfloreszenz-Analyse wird in Methanol : Aceton (1:1) an Zellen durchgeführt, indem man monoklonale Antikörper (National Cancer Institute USA) gegen HTLV-III p15 und p24 nimmt. Die mit oder ohne Penicillamin behandelten infizierten Zellen werden auf Toxoplasmose Objektträger befestigt. Nach 30 minütiger Behandlung mit Methanol-Aceton (1:1) bei Raumtemperatur werden die Objektträger in geschlossenen Plastikbehältern bei -20°C bis zum Gebrauch aufbewahrt. Die monoklonalen Antikörper werden zur Zellvermehrung hinzugefügt, bei Raumtemperatur in einer feuchten Kammer 1 Stunde inkubiert und mit PBS Pufferlösung enthaltend 0,25 % Triton X-100 zwei Stunden gewaschen. Die Zellen werden dann gegenüber goat-antimouse JgG (Capell Labs.) gebunden mit Fluoreszein (FITC) 1 Stunde ausgesetzt und mit PBS Pufferlösung enthaltend 0,25 % Triton X-100 über Nacht gewaschen. Die Objektträger werden mit 50 % Glycerol versehen, und die Zellfloreszenz wird mit einem Zeiss Floreszenz-Mikroskop beobachtet.

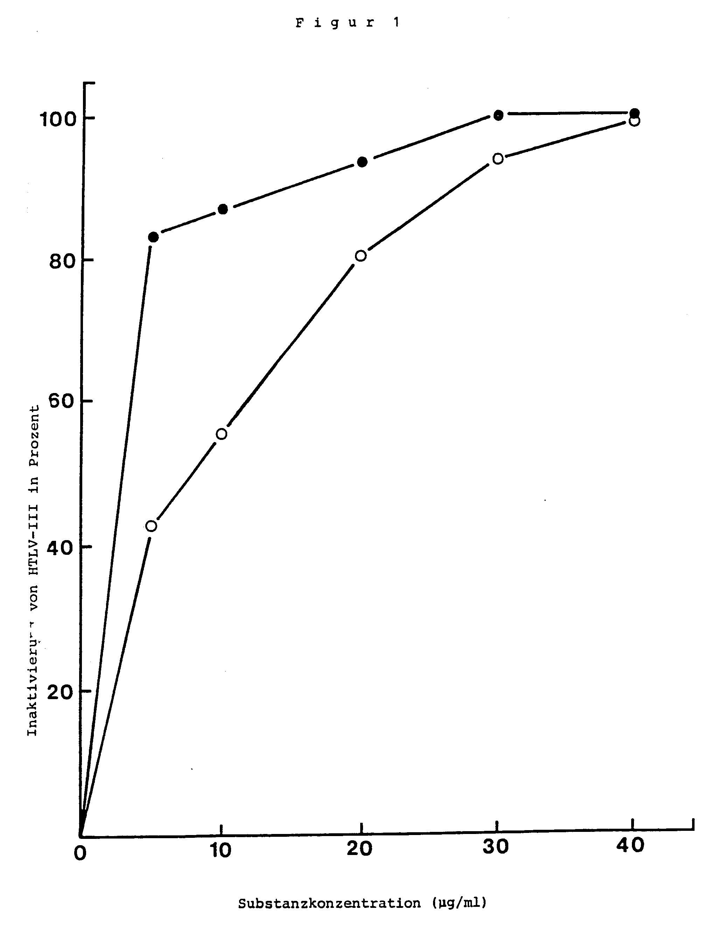

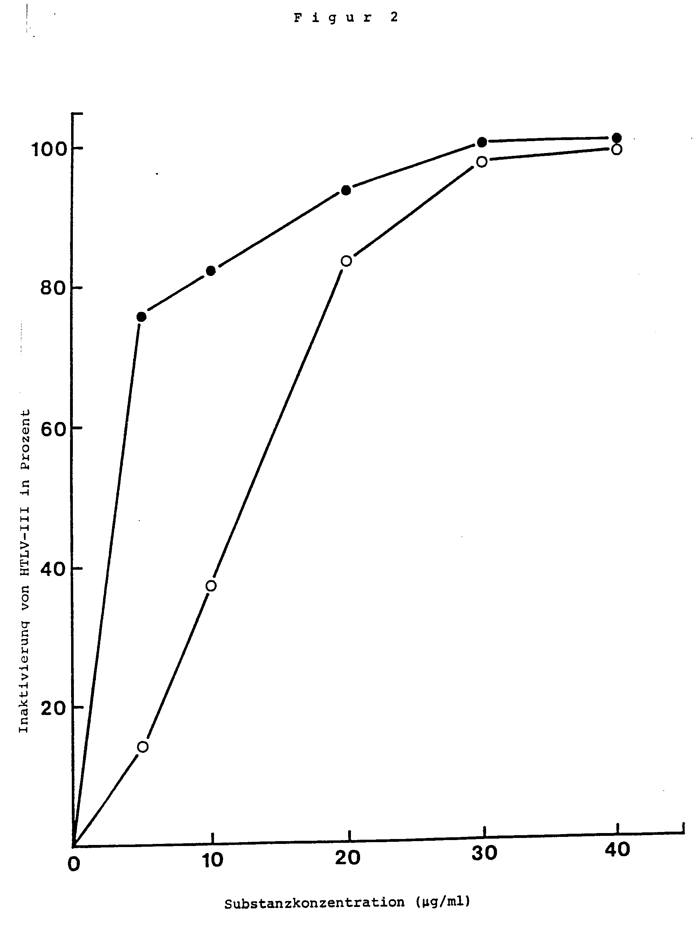

Der Effekt von D- und L-Penicillamin auf die Vermehrung von HTLV-III in H9 Zellen wird durch die Funktion der Substanzkonzentration bestimmt indem die Bildung der viralen Proteine p15 (Figur 1) und p24 (Figur 2) in einer Immunfloreszenz-Analyse mit monoklonalen Antikörpern bestimmt wird.

Figur 1 zeigt eine Konzentration in Abhängigkeit von der Verhinderung der Bildung von p15-Virusproteinen sowohl mit L-Penicillamin (schwarze Punkte) als auch mit D-Penicillamin (helle Punkte.) Bei niedrigeren Konzentrationen ist L-Penicillamin wirksamer als D-Penicillamin. Um eine Inaktivierung von 98,5 % bis 99,4 % zu erreichen, ist eine Substanzkonzentration von 40 µg/ml für beide Isomeren notwendig.

Figur 2 zeigt die Inaktivierung von HTLV-III durch D- und L-Penicillamin mit Hilfe der Immunfloreszenz-Analyse mit monoklonalen Antikörpern gegenüber dem viralen Protein p24. Beide Substanzen verhindern die p24 Bildung in gleicher Weise wie bei dem viralen Protein p15. Um eine völlige Inaktivierung der viralen Replikation zu erreichen, ist bei den Isomeren eine Konzentration von 40µg/ml nötig.

Um die Selektivität des Effektes auf die Replikation des HTLV-III Virus zu zeigen, kann die Wirkung von D- und L-Penicillamin auf das Wachstum von H9 Zellen geprüft werden. Die Wirkungen der beiden Substanzen auf infizierte und nicht infizierte Zellen wird in Tabelle 1 gezeigt.

T A B E L L E  1

Wirkung von D- und L-Penicillamin auf das Wachstum von infizierten und nicht infizierten H9 Zellen

| Versuch | Zellzahl/ml × $10^{-6}$ | | | |
| --- | --- | --- | --- | --- |
| | nicht infiziert | | infiziert | |
| | DP* | LP* | DP | LP |
| 1,24 | | | 0,18 | |
| µg/ml | | | | |
| 20 | | | 0,27 | 0,35 |
| 30 | | | 0,35 | 0,38 |
| 40 | | | 0,8 | 0,98 |
| 100 | 1.30 | 1.31 | | |
| 500 | 0.84 | 0.94 | | |
| 750 | 0.18 | 0.53 | | |

\* DP und LP bedeuten D- und L-Penicillamin
Zellzahl wird 4 Tage nach Versuchsbeginn bestimmt.

D-Penicillamin verhindert das Wachstum von nicht infizierten Zellen erst ab einer Konzentration über 100 ug/ml. Bei einer Konzentration von 500 ug/ml zeigt D-Penicillamin eine Inaktivierung des Zellwachstums von 32 %, bei der gleichen Konzentration zeigt L-Penicillamin eine Inaktivierung des Zellwachstums von ungefähr 24 % Substanzkonzentrationen von mehr als 500 ug/ml verhindern das Wachstum von nicht infizierten Zellen sehr stark.

Die Wirkung von D- und L-Penicillamin auf das Wachstum von infizierten Zellen zeigt in Tabelle 1 folgendes:

4 Tage nach der Infizierung mit dem HTLV-III Virus vermindert sich die Zahl der H9 Zellen von 1,24 x $10^6$ auf 0,18 x $10^6$. In Gegenwart von D- und L-Penicillamin findet sich mit steigender Konzentration eine beträchtliche Erhöhung der Zelldichte. Dies bebeutet, daß beide Substanzen einen Schutzeffekt auf T-Zellen haben.

Im allgemeinen soll die Penicillaminmenge im Blut der Patienten liegen: zwischen 10 und 400 vorzugsweise 30 und 300 beziehungsweise 40 bis 200 insbesondere 40 bis 100 beziehungsweise 40 bis 50 Mikrogramm pro ml Blut. Um diese Serumkonzentration beim Menschen zu erreichen, empfiehlt sich bei peroraler Applikation folgende Dosierung:

0,5 g bis 3 g, insbesondere 0,9 g bis 2,1 g, vorzugsweise, 1,5 g bis 2 g D-Penicillamin pro Tag beim Erwachsenen, wobei eine Dosierung von 3 g pro Tag nur über einen Zeitraum von ca. 1 Woche, von 2 g pro Tag über einen Zeitraum von 12 Monaten gegeben werden kann. Bei intravenöser Applikation empfiehlt sich beim Erwachsenen eine Gabe von 1 g bis 2 g wirksamer Substanz pro Tag, wobei eine Gabe von 0,5 bis 1,5 g, bevorzugt 1 g D-Penicillamin, in einer geeigneten Lösung verabreicht wird. Die empfohlenen Dosierungen sind bei Patienten im Kindesalter entsprechend zu reduzieren. Die Dosierung kann individuell auch in kleineren Dosen über den Tag erfolgen, beispielsweise bei peroraler Applikation 1 bis 6 mal täglich vorzugsweise 2-4 mal täglich 250 mg bis 500 mg D-Penicillamin. Eine Überdosierung von ca. 4 g D-Penicillamin über einen längeren Zeitraum soll vermieden werden.

Alle Mengenangaben in der Anmeldung beziehen sich auf die Penicillamin-Base. Bei Verwendung von Penicillamin-Salzen ist die entsprechende Menge jeweils entsprechend zu erhöhen.

Beispiel 1: Tabletten

300 g D-Penicillamin werden mit 0,25 g Äthylendiamintetraessigsäure-Dinatrium-Salz, 30 g Meisstärke und 5,25 g hochdisperser Kieselsäure gesiebt, in einem geeigneten Mischer gemischt und mit 120 g einer Lösung feucht granuliert, die aus 12 g Luviskol VA 64, 102 g Isopropanol und 6 g demineralisiertem Wasser besteht. Anschließend wird die feuchte Masse durch eine geeignete Granuliermaschine gegeben und getrocknet. Das trockene und gesiebte Granulat wird anschließend mit der äußeren Phase, die aus

| | |
|---|---|
| 90 g | Maisstärke |
| 50 g | Cellulose |
| 10 g | hochdisperser Kieselsäure und |
| 1,5 g | Magnesiumstearat |

besteht, versetzt und homogen gemischt. Danach wird diese Mischung zu Tabletten mit einem Gewicht von 500 mg verpresst.

Beispiel 2: Lackierte Tabletten

Die nach Beispiel 1 hergestellten Tabletten werden zum Schutz gegen die Einwirkung von Feuchtigkeit und Luftsauerstoff und zur Überdeckung des unangenehmen Geschmacks und Geruchs des D-Penicillamins mit einem magenlöslichen Schutzfilm überzogen. Der Schutzfilm kann in einem Dragierkessel oder einer geeigneten Wirbelbett-Anlage auf die Tabletten aufgetragen werden.

Auf 500 g = 1000 Tabletten werden 87,5 ml einer Suspension aufgetragen, die sich wie folgt zusammensetzt:

| | Angabe in % Gew./Gew. |
|---|---|
| Äthylcellulose [*] | 2 % |
| Hydroxypropylcellulose [*] | 1 % |
| Polyäthylenglykol 5/6000 | 2,5 % |
| Glycerin | 0,5 % |
| Titandioxid | 3,5 % |
| Talkum | 1,5 % |
| Isopropanol | 44,5 % |
| 1,1,1-Trichloräthan | 44,5 % |
| | 100,0 % |

[*] Als Filmbildner eignen sich verschiedene Äthyl- und Hydroxypropylcellulosen, die beispielsweise unter der Handelsbezeichnung Ethocel und Klucel durch die Firmen Dow, Hercules und Syntana zu beziehen sind.

Beispiel 3: Herstellung von Gelatine-Steck-Kapseln mit D-Penicillamin, HCL

185 g     D-Penicillamin, HCl
3 g     hochdisperse Kieselsäure
9 g     tri-Calciumphosphat

werden gemischt und mit 60 g einer Lösung, die aus 5 % Hydroxypropylmethylcellulose, 75 % Gew./Vol. Äthanol und 20 % demineralisiertem Wasser besteht, in bekannter Weise granuliert. Das trockene Granulat wird in Einzelmengen von 200 mg in Gelatine-Steck-Kapseln abgefüllt. 1 Kapsel enthält 185 mg D-Penicillamin, HCl.

Beispiel 4: Herstellung von D-Penicillamin-Trockenampullen

123 g D-Penicillamin.HCl (entsprechen 100 g D-Penicillamin) werden mit destilliertem Wasser zu einem Gesamtvolumen von 500 ml unter schwachem Erwärmen auf dem Wasserbad gelöst. Die Lösung wird durch einen Entkeimungsfilter gegeben und in Portionen á 5 ml in geeignete Stech-Ampullen abgefüllt. Der wässrige Ampulleninhalt wird nach allgemein bekannten Methoden, zum Beispiel der spin-freezing-Methode eingefroren und danach lyophilisiert. Nach Beendigung der Lyophilisation werden die Steck-Ampullen unter sterilen Kautelen mit Gummistopfen und Aluminium-Kappen verschlossen.

Um aus der Trocken-Ampulle eine injektionsfertige Lösung herzustellen, wird das Lyophilisat in 10 ml sterilem Lösungsmittel gelöst. Das Lösungsmittel besteht aus einer wässrigen Lösung von Tris-(hydroxymethyl)-aminomethan (Trometamol) oder einer anderen geeigneten organischen Base, wobei die Base in einer solchen Menge einzusetzen ist, daß die injektionsfertige Lösung einen pH-Wert von 4,0 bis 4,5 besitzt.

1 Trocken-Ampulle enthält 1,23 g D-Penicillamin.HCl entsprechend 1,0 g D-Penicillamin.

Beispiel 5: Herstellung von D-Penicillamin-Suppositorien

300 g D-Penicillamin werden in 1700 g geschmolzene Suppositorienmasse (zum Beispiel Hartfett DAB 7) eingearbeitet und in bekannter Weise in Formen für 2,0 g Suppositorien ausgegossen.

1 Suppositorium enthält 300 mg D-Penicillamin

Beispiel 6: Herstellung einer D-Penicillamin-Salbe

50 g D-Penicillamin werden in 660 g demineralisiertem Wasser gelöst. Die Lösung wird unter ständigem Rühren in eine Schmelze eingetragen, die aus 125 g Emulsan MD[1], 14 g Lanette E[2] und 15 g Cetiol V[3] besteht. Der RührVorgang wird so lange fortgesetzt, bis eine Salbe entsteht, in der der Wirkstoff homogen verteilt ist. In 100 g Salbe sind 5 g D-Penicillamin echt gelöst.

Beispiel 7: Herstellung einer Inhalationslösung

100 g D-Penicillamin werden mit destilliertem Wasser, in dem zuvor unter Stickstoffbegasung 0,5 g Äthylendiamintetraessigsäure-Dinatrium-Salz und 0,5 g Natriummetabisulfit in Lösung gebracht wurden, zu einem Gesamtvolumen von 1000 ml unter schwachem Erwärmen auf dem Wasserbad gelöst. Die Lösung wird durch einen Entkeimungsfilter gegeben und unter Stickstoffbegasung in Flaschená 50 ml abgefüllt. 1 ml Inhalationslösung enthält 50 mg D-Penicillamin.

Beispiel 8: Herstellung von Gelatine-Steck-Kapseln mit D-Penicillamin und Salicylamid

185 g     D-Penicillamin.HCl
7,5 g     Mannit
500 g     Salicylamid

werden gemischt und in bekannter Weise mit 150 g einer Lösung granuliert, die aus 5 % Hydroxypropylmethylcellulose, 75 % Gew./Vol. Äthanol und 20 % demineralisiertem Wasser besteht. Das trockene Granulat wird in Einzelmengen von 700 mg in Gelatine-Steck-Kapseln abgefüllt. 1 Kapsel enthält 185 mg D-Penicillamin.HCl und 500 mg Salicylamid.

**Patentansprüche**

**1.** Verwendung von D-Penicillamin und deren Säureadditionssalzen zur Herstellung eines Arzneimittels gegen das Acquired Immune Deficiency Syndrom (AIDS).

**Claims**

**1.** The use of D-penicillamine and acid addition salts thereof for the production of a medicament for the treatment of Acquired Immune Deficiency Syndrome (AIDS).

**Revendications**

**1.** Utilisation de la D-pénicillamine et de ses sels d'addition d'acide pour la préparation d'un médicament contre le syndrome immuno-déficitaire acquis (SIDA).

*) 1) Gemisch    von   Mono-   und   Diglyceriden    der   Palmitin-    und   Stearinsäure

2) Natriumcetylstearylsulfat

3) Ölsäuredecylester

Figur 1

**F i g u r  2**